(19) Europäisches Patentamt
European Patent Office
Office européen des brevets

(11) Veröffentlichungsnummer : **0 013 727**
**B1**

(12) **EUROPÄISCHE PATENTSCHRIFT**

(45) Veröffentlichungstag der Patentschrift : **07.04.82**

(21) Anmeldenummer : **79105050.3**

(22) Anmeldetag : **10.12.79**

(51) Int. Cl.³ : **C 07 D233/72**, C 07 D233/74, C 07 D233/76, C 07 D233/78, C 07 D233/86, C 07 D403/04, C 08 G 18/78, C 08 G 18/80, C 09 D 3/72, C 09 J 3/16

(54) **Verfahren zur Herstellung von (Thio)hydantoinen und ihre Verwendung als hitzebeständige Überzugsmaterialien, Folien, Pulver, Kleber, Formkörper, Lacke und Fasern.**

(30) Priorität : **23.12.78 DE 2855937**

(43) Veröffentlichungstag der Anmeldung : **06.08.80 (Patentblatt 80/16)**

(45) Bekanntmachung des Hinweises auf die Patenterteilung : **07.04.82 Patentblatt 82/14**

(84) Benannte Vertragsstaaten : **AT DE FR GB IT**

(56) Entgegenhaltungen :
**DE - A - 1 570 552**
**DE - A - 2 318 198**
**DE - A - 2 318 205**

(73) Patentinhaber : **BAYER AG**
**Zentralbereich Patente, Marken und Lizenzen**
**D-5090 Leverkusen 1, Bayerwerk (DE)**

(72) Erfinder : **Lewalter, Jürgen, Dr.**
**Bergstrasse 88**
**D-5068 Odenthal (DE)**
Erfinder : **Rottmaier, Ludwig, Ing.-grad.**
**Bergstrasse 85**
**D-5068 Odenthal (DE)**
Erfinder : **Merten, Rudolf, Dr.**
**Berta-von-Suttner-Strasse 55**
**D-5090 Leverkusen 1 (DE)**
Erfinder : **Dünwald, Willi, Dr.**
**Geschw.-Scholl-Strasse 16**
**D-5090 Leverkusen 1 (DE)**
Erfinder : **Schulte, Bernhard, Dr.**
**Südwall 80 A**
**D-4150 Krefeld (DE)**

**EP 0 013 727 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von (Thio)hydantoinen und ihre Verwendung als hitzebeständige Überzugsmaterialien, Folien, Pulver, Kleber, Formkörper, Lacke und Fasern

Die Erfindung betrifft ein Verfahren zur Herstellung von Kondensaten mit mindestens einer Hydantoin- oder Thio-hydantoinring im Molekül aus α-Hydroxycarbonsäurederivaten und/oder α-Sul hydrylcarbonsäurederivaten und organischen Isocyanaten sowie deren Verwendung als biochemisch Wirkstoffe bzw. zur Herstellung hitzeresistenter Beschichtungsmittel und Beschichtungen.

Verfahren zur Herstellung von Hydantoinen (Am. Chem. J. 45/383 (1911)) bzw. Polyhydantoin (BE 678 282) sind bekannt und inzwischen in den verschiedenartigsten Variationen und Ausführungs formen in den unterschiedlichsten Lösungsmitteln beschrieben worden. So werden gemäß der Lehre de DOS 2 318 205 und 2 318 198 hydantoinhaltige Verbindungen unter Einsatz von α-Halogencarbonsäure bzw. den entsprechenden Amiden hergestellt, wobei aber bei diesen Reaktionen Halogenwasserstoffsäu re abgespalten wird, die nur schwierig aus der Reaktionsmischung entfernt werden und Korrosions probleme mit sich bringen kann.

Niedermolekulare Hydantoine werden bevorzugt im Pharma- und Pflanzenschutzbereich, höhermo lekulare Hydantoine beispielsweise im wärmebeständigen Beschichtungsmittelsektor verwendet (fran: Pat. 1 484 694).

Überraschenderweise wurde nun gefunden, daß durch Umsetzung von mindestens 2 Mol gegebe nenfalls maskierter organischer Iso(thio)cyanate mit einem Mol eines α-Hydroxy- und/oder α-Sul hydrylcarbonsäurederivates mit der allgemeinen Formel I :

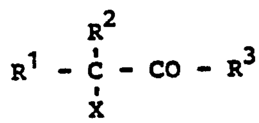 (

in der

X für eine OH- und SH-Gruppe steht,

$R^1$ und $R^2$, unabhängig voneinander Wasserstoff, ein Halogenatom, eine CN-Gruppe, eine COF Gruppe mit R = Hydroxyl-, Amino-, Alkyl-amino-, Dialkylamino-, Alkoxy- oder Aroxy-gruppe, Wasserstof einen aliphatischen, cycloaliphatischen, aliphatisch-aromatischen, aromatischen oder heterocyclische Rest ; ein aliphatischer Rest, ein cycloaliphatischer Rest, ein aliphatisch-aromatischer Rest, ei aromatischer Rest, ein heterocyclischer Rest, die jeweils noch gegebenenfalls mit Halogen-, $-NO_2-$ $-COR^4-$, $-CN-$, $-CO-$, $-OH$-Gruppen mit $R^4 = R$ substituiert oder auch gegebenenfalls gemeinsar bzw. jeder für sich mit $R^3$ als Glieder entsprechender cyclischer organischer Reste miteinander verknüp sein können und

$R^3$ eine Hydroxyl-, Amino-, Alkylamino, Dialkylamino-, Alkoxy- oder Aroxygruppe bedeuten, be Temperaturen von −20 bis 500 °C, vorzugsweise von 0 bis 450 °C, mindestens einen Hydatoinring bzw Thiohydantoinring enthaltende Verbindungen in guten Ausbeuten erhalten werden.

Der Umsatz von α-Hydroxy- und/oder α-Sulfhydrylcarbonsäurederivaten mit organischen Isocyante liefert bekanntlich bevorzugt die entsprechenden Oxazolidindione, deren heterocyclisches Gerüst selbs bei höherer Temperatur relativ beständig ist. Überraschenderweise führt jedoch der Einsatz vo mindestens 2 Val Isocyanat pro Äquivalent eines α-Hydroxy- und/oder α-Sulfhydrylcarbonsäurederivate zur Hydantoinringbildung.

Der glatte Ablauf der in mehreren Stufen verlaufenden Reaktion, der bei Einsatz von Polyisocyanate auch einen Aufbau von Polymeren ermöglicht, war nicht zu erwarten, da jede Stufe, speziell in Gegenwa überschüssiger Isocyanatmengen, zu einer Vielzahl komplizierter Stoffgemische reagieren kann un speziell die intermediären 2,4-Oxazolidindione als relativ stabile, temperaturbeständige Heterocycle beschrieben werden (R.C. Elderfield, Heterocyclic Compounds, Vol. 5 (1957) 411 ff). Im Zuge de erfindungsgemäßen Umsetzung werden nur beliebig verfügbare Komponenten verwendet und somit di meist zahlreichen Vorstufen der bisher bekannten Hydantoinherstellungsverfahren vermieden.

Die erfindungsgemäße Reaktion kann formal durch die nachfolgende Gleichung beispielha wiedergegeben werden ;

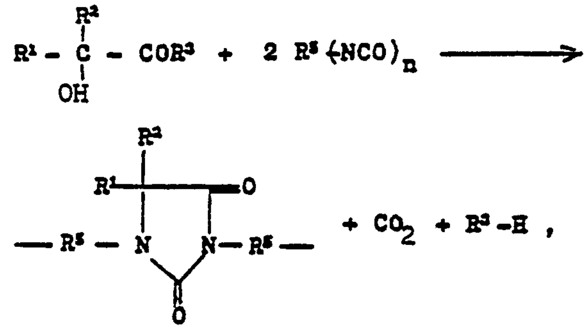

wobei die Reste :

$R^1$ bis $R^3$ die oben angegebene Bedeutung haben,

$R^5$ der gegebenenfalls substituierte aliphatische, cycloaliphatischen, aliphatisch-aromatische, aromatische oder heterocyclische Rest eines organischen Isocyanates und

n eine ganze Zahl von 1-4, vorzugsweise 1-2 sein soll.

Je Mol des α-Hydroxy- und/oder α-Sulfhydrylcarbonsäurederivates können 2 bis 8 Val, vorzugsweise 2 bis 4 Val Isocyanat eingesetzt werden, doch wird der Verlauf der Reaktion nicht durch überschüssiges Isocyanat gesört. Für

n = 1 werden monomolekulare Hydantoine der allgemeinen Formel

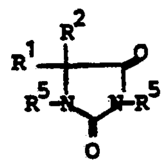

und für

n > 1 werden in Abhängigkeit von den stöchiometrischen Verhältnissen der Ausgangsmaterialien oligomere oder polymere Hydantoine mit der wiederkehrenden Struktureinheit der allgemeinen Formel

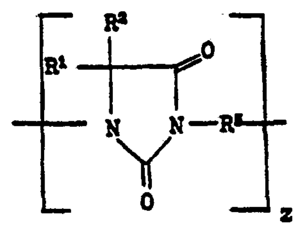

erhalten, wobei die Reste

$R^1$, $R^2$ und $R^5$ die oben angegebene Bedeutung haben und

z für eine ganze Zahl von 2 bis 2 000, vorzugsweise 2 bis 1 000 steht.

Werden je Mol des α-Hydroxy- und/oder α-Sulfhydrylcarbonsäurederivates mehr als 1, vorzugsweise 1,5 bis 2,0 Mol eines Diisocyanates eingesetzt, so entstehen Polyisocyanate mit der gegebenenfalls wiederkehrenden Struktureinheit :

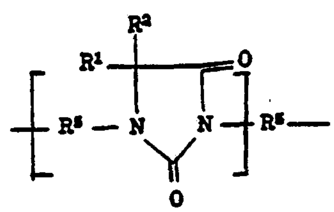

die gegebenenfalls über $R^5$-Reste verknüpft ist, und gegebenenfalls verkappte NCO-Endgruppen enthält.

Die Reste $R^1$, $R^2$ und $R^5$ haben die oben angegebene Bedeutung.

Die Hydantoinisocyanate dieser Reihe sind als Komponenten und Vernetzer, beispielsweise in Polyestern, Polyethern Polyurethanen, Polyhydantoinen, Polyesteramidimiden, Polyamidimiden und Polyimiden geeignet.

Die Hydantoine können über die charakteristischen IR-Banden der Hydantoine identifiziert werden. Die höhermolekularen Hydantoine besitzen in 30 gew.-%igen Lösungen mit beispielsweise Acetophenonen, Butyrolacton, Kresol, Benzylalkohol oder Benzoesäurealkylestern bei 25 °C Lösungsviskositäten von 50 bis 200 000 mPas, vorzugsweise von 500 bis 100 000 mPas.

Vorzugsweise werden als α-Hydroxy- und/oder α-Sulfhydrylcarbonsäurederivate Verbindungen der allgemeinen Formel verwendet

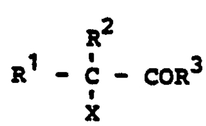

in der

X eine OH- oder eine SH-Gruppe,

$R^1$ und $R^2$ gleich oder verschieden, Wasserstoff, ein Halogenatom, eine —CN-Gruppe, eine —COR-Gruppe mit R = Hydroxyl-, Amino-, Alkylamino-, mit $C_1$-$C_{20}$, Dialkylamino- mit jeweils $C_1$-$C_{20}$, Alkoxy- mit $C_1$-$C_{20}$ oder Aroxygruppe mit $C_6$-$C_{12}$, Wasserstoff, einem aliphatischen Rest mit $C_1$-$C_{20}$, aliphatisch-aromatischen Rest mit $C_7$-$C_{20}$, aromatischen Rest mit $C_6$-$C_{20}$ oder heterocyclischen Rest mit 4-16 Ringgliedern,

$R^1$ und $R^2$ weiterhin ein aliphatischer Rest mit $C_1$-$C_{20}$, ein aliphatisch-aromatischer Rest mit $C_7$-$C_{20}$, ein aromatischer Rest mit $C_6$-$C_{20}$, ein heterocyclischer Rest mit 4-16 Ringgliedern, die jeweils noch gegebenenfalls mit Halogen-, $NO_2$-, $COR^4$-, CN-, —CO-, OH-Gruppen mit $R^4$ = R substituiert sein können und

$R^3$ eine Hydroxyl-, Amino-, Alkylamino mit $C_1$-$C_{20}$ Dialkylamino mit jeweils $C_1$-$C_{20}$, Alkoxy mit $C_1$-$C_{20}$ oder Aroxygruppe mit $C_6$-$C_{12}$ bedeuten.

Die Reste $R^1$ und $R^2$ können bevorzugt Wasserstoff, Fluor, Chlor, Brom, ein Methan-, Ethan-, Hexan-, Cyclohexan-, Propen-, Benzol-, Toluol-, Piperidin-, Morpholin-, Imidazol-, Formylalkyl-, Alkylcarbonylalkyl-, bzw. Arylcarbonylalkyl-, ein Alkoxycarbonylalkyl-, Aroxycarbonylalkylrest sein und gegebenenfalls gemeisam oder jeweils für sich zusammen mit dem Rest $R^3$ einen bis zu 8 Ringgliedern enthaltenden Ring bilden.

$R^2$ kann vorzugsweise ein —$CH_2$—COR-Rest sein mit R wie oben definiert, und der gegebenenfalls gemeinsam mit den Resten $R^1$ bzw. $R^3$ einen bis zu 8 Ringgliedern enthaltenden Ring bilden kann.

Besonders bevorzugt sind der Rest $R^1$ Wasserstoff, ein Alkyl- mit $C_1$-$C_7$ oder Arylrest mit $C_6$-$C_{10}$, der Rest $R^3$ ein Alkoxy mit $C_1$-$C_7$ oder Aroxy-Rest mit $C_6$-$C_{10}$ und der Rest $R^2$ ein Alkoxycarbonylalkyl-$C_3$-$C_{10}$ bzw. Aroxycarbonylalkylrest mit $C_8$-$C_{12}$.

Als Beispiele bevorzugter α-Hydroxy- und/oder α-Sulfhydrylcarbonsäurederivate sind die Säure-, Ester- bzw. Amidderivate folgender Verbindungen aufzuführen :

Glykolsäure,
Milchsäure,
α-Hydroxypropionsäure,
α-Hydroxybuttersäure,
Atrolactinsäure,
Mandelsäure,
Benzilsäure,
α-Hydroxy-β-phenylpropionsäure,
α-Hydroxy-γ-oxo-γ-phenylbuttersäure,
α-Hydroxy-lävulinsäure
α-Hydroxy-γ-oxo-buttersäure,
α-Hydroxy-β-oxo-buttersäure,
2,4-Dihydroxy-5-oxo-1,2-dihydrofuran,
3-Hydroxy-2-oxo-5-phenyl-tetrahydrofuran,
Glycerinsäure,
Tartronsäure,
Apfelsäure,
Weinsäure/Traubensäure,
Citronensäure,
Ascorbinsäure,
α-Hydroxy-oxo-bernsteinsäure, usw.

sowie die entsprechenden Thioanaloga genannt.

Als organische Isocyanate können Mono- und/oder Polyiso-(thio)-cyanate verwendet werden.

Als Monoisocyanate im Sinne der Erfindung werden aliphatische und aromatische, gegebenenfalls durch Heteroatome substituierte Verbindungen mit einer NCO-Gruppe im Molekül eingesetzt, z.B. Alkylisocyanate wie Äthyl-, Methyl-, Butyl-, Dodecyl- und Stearylisocyanat, aromatische, gegebenenfalls substituierte Monoisocyanate wie Phenyl-, Tolyl-, Isopropyl-, Nonylisocyanat, Nitro-, Alkoxy-, Aroxy-, Chlor-, Dichlor-, Trichlor-, Tetrachlor-, Pentachlor-, Benzyl-, Brom-phenyl-isocyanat oder Isocyanatobenzoesäureester, -phthalsäureester, -isophthalsäureester, Isocyanatobenzonitril, cycloaliphatische Isocyanate wie Cyclohexylisocyanat und ungesättigte Isocyanate wie Allyl-, Oleyl-, Cyclohexenylisocyanat.

Als erfindungsgemäß einzusetzende Ausgangskomponenten kommen ebenso aliphatische, cycloaliphatische, araliphatische, aromatische und heterocyclische Polyisocyanate in Betracht (vgl. Annalen 562, Seiten 75 bis 136), beispielsweise Äthyllen-diisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanato-methylcyclohexan (DAS 1 202 785), 2,4- und 2,6-Hexahydrotoluylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4''-triisocyanat, Polyphenyl-polymethylen-polyisocyanate, wie sie durch Anilin-Formaldehyd-Kondensation und anschließende Phosgenierung erhalten und z.B. in den britischen Patentschriften 874 430 und 848 671 beschrieben werden, perchlorierte Arylpolyisocyanate, wie sie z.B. in der deutschen Auslegeschrift 1 157 601 beschrieben werden, Carbodiimidgruppen aufweisende Polyisocyanate, wie sie in der deutschen Patentschrift 1 092 007 beschrieben werden, Diisocyanate, wie sie in der US-Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der US-Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift 1 101 394, in der britischen Patentschrift 889 050 und in der französischen Patentschrift 1 017 514 beschrieben werden, durch Telomerisationsreaktionen hergestellte Polyisocyanate, wie sie z.B. in der belgischen Patentschrift 723 640 beschrieben werden, Estergruppen aufweisende Polyisocyanate, wie z.B. in den britischen Patentschriften 956 474 und 1 072 956, in der US-Patentschrift 3 567 763 und in der deutschen Patentschrift 1 231 688 genannt werden, Umsetzungsprodukte der obengenannten Isocyanate mit Acetalen gemäß der deutschen Patentschrift 1 072 358.

Es ist auch möglich, die bei der technischen Isocyanatherstellung anfallenden Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Vorzugsweise eignen sich Isocyanate der allgemeinen Formel

$$R^5(—NCO)_n,$$

in denen

$R^5$ für einen, gegebenenfalls mit Halogen, Alkyl- mit $C_1$-$C_{10}$ und/oder Arylgruppen mit $C_6$-$C_{12}$ substituierten Alkylrest mit 1-20 C-Atomen, einen Arylrest mit 6-12 C-Atomen, einen Cycloalkylrest mit 5-12 C-Atomen, einen Alkyl-Arylrest mit 7-20 C-Atomen und einen Heteroatome wie N und/oder O und/oder S enthaltenden Aryl- oder Cycloalkylrest mit 5-12 Ring C-Atomen steht,

n ist eine ganze Zahl von 1-4, vorzugsweise 1-3, besonders bevorzugt 2.

Besonders bevorzugt sind aliphatische Reste mit 2-12 C-Atomen oder Arylreste wie Phenyl, Tolyl, Naphthyl, Diphenylmethan- und Diphenylätherreste.

Bevorzugt verwendet werden die technisch leicht zugänglichen Gemische aus Toluylen-diisocyanaten, m-Phenylendiisocyanat, sowie phosgenierte Kondensate aus Anilin und Formaldehyd mit Polyphenylen-methylen-struktur und die symmetrischen Verbindungen 4,4'-Diisocyanatodiphenylmethan, 4,4'-Diisocyanatodiphenyläther, p-Phenylendiisocyanat und 4,4'-Diisocyanatodiphenyldimethylmethan sowie Isophorondiisocyanat und Hexamethylendiisocyanat.

Die Isocyanate können in freiter Form, ferner zum Teil oder vollständig auch in Form ihrer beim Umsatz mit reaktiven Wasserstoff enthaltenden Verbindungen zugänglichen und unter den Reaktionsbedingungen als Isocyanat-Abspalter reagierenden Derivate eingesetzt werden.

Vorzugsweise werden als Abspalter die Additionsprodukte an Lactamen, Oximen und CH-aciden Verbindungen sowie die aus aliphatischen und aromatischen Mono- und Polyhydroxy-Verbindungen erhaltenden Carbamidsäureester, beispielsweise der allgemeinen Formel

$$R^5(-NH-\underset{\underset{O}{\|}}{C}-O-M)_n \quad \text{bzw.} \quad \left[-\underset{\underset{O}{\|}}{C}-NH-R^5-NH-\underset{\underset{O}{\|}}{C}-O-Q-O-\right]_y$$

eingesetzt, worin

$R^5$ und n die oben angegebene Bedeutung haben und

M der organische Rest einer Monohydroxyverbindung bzw.

Q der organische Rest einer bis- oder trisfunktionellen Hydroxyverbindung, vorzugsweise M und Q, gleich oder verschieden, ein aliphatischer Rest mit 1-10 C-Atomen, ein cycloaliphatischer Rest mit 5-12 C-Atomen, ein aliphatisch-aromatischer Rest mit 7-12 C-Atomen und/oder ein aromatischer Rest mit 6-12 C-Atomen ist, die jeweils noch mit Alkyl- mit $C_1$-$C_{12}$ und/oder Arylgruppen mit $C_6$-$C_{12}$ substituiert sein können, und

y für eine ganze Zahl von 1 bis 1000, vorzugsweise 1 bis 100 steht.

Als Beispiele seien die Carbamidsäureester aus Phenol, isomeren Kresolen, deren technischen Gemischen und ähnlichen aromatischen Hydroxylverbindungen, aliphatische Monoalkohole wie Methanol, Äthanol, Propanol, Isopropanol, Butanol, Isobutanol, Cyclohexanol, Allylalkohol, Benzylalkohol und

aliphatische Di- oder Polyole wie Ethynelenglykol und Trimethylolpropan, Glycerin und/oder Trishydroxyalkylisocyanuraten usw., weiterhin die Additionsprodukte mit Pyrrolidon- (2), Caprolactam, Butanonoxim, Malonester, Acetessigester und Acetophenon aufgeführt.

Die Isocyanat-Abspalter können als solche eingesetzt oder erst in situ durch Umsetzung mit den entsprechenden Reaktanten erzeugt werden.

Anstelle der genannten (Poly)Isocyanate können auch die analogen (Poly)-Isothiocyanate als Ausgangsmaterialien benutzt werden.

Die erfindungsgemäß als Blockierungs- wie auch Lösungsmittel besonders bevorzugten Hydroxyalkyläther sind beispielhaft Verbindungen der allgemeinen Formel

$$R^6—(OR^7)_p—OH,$$

in der

$R^6$ ein gegebenenfalls substituierter aliphatischer Rest mit $C_1$-$C_{20}$, vorzugsweise $C_1$-$C_8$, cycloaliphatischer Rest mit $C_5$-$C_{12}$, vorzugsweise $C_5$-$C_8$, aliphatisch-aromatischen Rest mit $C_7$-$C_{16}$ oder aromatischer Rest mit $C_6$-$C_{14}$, der z.B. mit Alkoxy-, Aroxy- oder Hydroxy-Gruppen substituiert sein kann,

$R^7$ ein aliphatischer Rest mit 2-20 C-Atomen, und

p eine ganze Zahl von 1-100, bevorzugt 1-4, bedeuten.

Vorzugsweise werden erfindungsgemäß solche Hydroxyalkyläther eingesetzt, die pro Molekül eine Hydroxy-Gruppe enthalten und in denen $R^7$ ein Rest mit zwei C-Atomen in der Kette, die beispielsweise durch Alkyl-Gruppen substituiert sein können, bedeutet, z.B. die Methyl-, Isopropyl-, Cyclohexyl-, Benzyl-, Phenyl- und Methoxy-äthyl-äthylenglykol- und -propylenglykol- bzw.-diäthylenglykol- und -dipropylenglykol-monoäther.

Der erfindungsgemäße Umsatz der α-Hydroxy- bzw. α-Sulfhydryl-Carbonsäurederivate mit den organischen Isocyanaten zu den Hydantoinen bzw. Hydantoingruppen enthaltenden Verbindungen kann in Lösungsmitteln, die unter den Reaktionsbedingungen nicht reagieren oder lockere, weiterreagierende Additionsverbindungen bilden, oder in Substanz, oder in einem Überschuß einer der Reaktionskomponente durchgeführt werden.

Neben den aufgeführten Blockierungsmitteln eignen sich als Lösungsmittel Kohlenwasserstoffe, Halogenkohlenwasserstoffe, Alkohole, Ester, cyclische Ester, Ketone, Ether, substituierte Amide, Nitrile; Beispiele dafür sind Xylole, O-Dichlorbenzol, Benzylalkohol, Phenoxyethanol, Acetophenon, Cyclohexanon, Ethylenglykolbutylether, Diethylenglykolmethylether, Propylencarbonat, ε-Caprolactam, Dimethylsulfoxid, Glykolmonomethyletheracetat, γ-Butyrolacton, ε-Caprolacton, Benzoesäurealkylester, N-Methylpyrrolidon, Dimethylformamid, Dimethylacetamid, Benzonitril und andere sowie deren Gemische.

Als Verschnittmittel sind außerdem aliphatische und aromatische Kohlenwasserstoffe wie Cyclohexan, Xylol, Toluol und deren technische Gemische wie Solvesso 100 und Solventnaphtha geeignet.

Es müssen jedoch keine Lösungsmittel, gegebenenfalls können stöchiometrische Blockmittelmengen verwendet werden.

Zur Durchführung der erfindungsgemäßen Verfahren werden die Reaktionskomponenten, mit oder ohne Lösungsmittel und/oder Blockierungsmittel, einige Minuten bis zu mehreren Stunden bei Temperaturen von −20 °C bis 500 °C, bevorzugt 0 °C bis 450 °C, gehalten. Der Reaktionsverlauf kann über die Gasentwicklung und die IR-Spektren verfolgt werden.

Die Acidität saurer Lösungsmittel wie der Phenole bzw. Kresole kann im Rahmen der Reaktionsführung zu verkürzten Reaktionszeiten führen. In inerten Medien oder in Schmelzansätzen können beispielsweise Carbonsäuren mit hinreichendem Schmelz- bzw. Siedepunkt wie Essigsäure, Benzoesäure, Bernsteinsäure, Benzoldicarbonsäuren, Butantetracarbonsäure, Trimellithsäure bzw. deren Anhydride gegebenenfalls auch als einbaufähige Katalysatoren in Mengen von 0,1 bis 40, vorzugsweise von 1 bis 10 Prozent in Val, bezogen auf ein Val Isocyanat, mitverwendet werden.

Zur Beschleunigung der ablaufenden Reaktionen sind die aus der Isocyanatchemie bekannten Katalysatoren wie Basen, beispielsweise Triethylamin, N-Methylmorpholin, Endoethylenpiperazin, wie Säuren, beispielsweise p-Toluol-sulfonsäure, wie Metalle, insbesondere von Eisen, Blei, Zink, Zinn, Kupfer, Kobalt, Titan, Mangan, beispielsweise Titantetrabutylat, Titanaminoalkohol, Eisenacetylacetonat, Dibutylzinnlaurat, Bleiacetat, Zinkoctoat oder Phosphorverbindungen wie Trialkylphosphin, zu verwenden.

Zuweilen ist es vorteilhaft, die Reaktion in mehreren Stufen durchzuführen. Durch stufenweise Temperaturführung und/oder durch stufenweise oder schrittweise Zugabe, gegebenenfalls verschiedener Isocyanate bzw. Isocyanatgemische kann ein definierter Aufbau der erfindungsgemäßen Verfahrensprodukte erreicht werden.

So kann beispielsweise in einer ersten Stufe, gegebenenfalls in einem Lösungs- und/oder Blockmittel ein Addukt oder Kondensat hergestellt werden, das dann bei höherer Temperatur, eventuell unter Verdampfung des Lösungsmittels, gegebenenfalls nach Zusatz von Blockierungsmittel unter Cyclisierung und/oder Kettenverlängerung und/oder Vernetzung in das gegebenenfalls hochmolekulare Kondensationsprodukt übergeht. Wird dieses dann zur Beschichtung verwendet, so kann es auch aus Schmelzen oder wäßrigen Systemen oder als Pulver appliziert werden.

Mitunter wird die Umsetzung zweckmäßigerweise unter einem inerten Schutzgas wie $N_2$ oder Argon durchgeführt.

Schließlich kann die erfindungsgemäße Reaktion in kontinuierlicher oder diskontinuierlicher Ausführung oder gegebenenfalls in Autoklaven unter Druck zum Zwecke einer höheren Reaktionstemperatur erfolgen.

Im allgemeinen werden entsprechend der erfindungsgemäßen Reaktion, vorteilhafterweise pro Mol des α-Hydroxy- und/oder α-Sulfhydrylcarbonsäurederivates mindestens 2 Val bis 8 Val, vorzugsweise 2 bis 4 Val Isocyanat eingesetzt.

Die Aufarbeitung der niedermolekularen Reaktionsprodukte kann nach gängigen Methoden wie z.B. Kristallisation erfolgen.

Eine weitere Ausführung der erfindungsgemäßen Reaktion besteht darin, daß gegebenenfalls in situ beispielsweise mehrwertige Amine, mehrwertige Alkohole, beispielsweise Ethylenglykol, Dipropylenglykol, Trimethylolpropan, Glycerin, Trishydroxyethylisocyanurat und/oder die Kombination aus Cyanursäuretriarylestern und Polyolen und/oder gegebenenfalls mehrwertigen Carbonsäuren und/oder deren Ester und/oder deren Anhydride, beispielsweise auf Basis der genannten Polycarbonsäuren, vorzugsweise der Phthalsäure, Isophthalsäure, Terephthalsäure, Trimellithsäure, Pyromellithsäure, Butantetracarbonsäure usw., und/oder polyfunktionelle, vorzugsweise bis-funktionelle α-Aminocarbonsäurederivate gemäß der belgischen Patentschrift 678 282 oder dem US-Patent 3 397 253 und/oder weitere Polyisocyanate bzw. Polyisocyanat-Abspalter, bzw. die diesen entsprechenden Polyamine sowie gegebenenfalls unter Zusatz von Hilfs- und Zusatzstoffen wie ε-Caprolactam, ε-Caprolacton usw. mitverwendet und in der üblichen Weise zu linearen und/oder verzweigten Kunststoffen mit z.B. Hydantoin-, Ester-, Carbamidester-, Amid- und/oder Imid-Gruppen von guter Löslichkeit, höherer Temperaturbeständigkeit, guter Elastizität und gutem Hitzeschockverhalten umgesetzt werden können.

In einer speziellen Variante des erfindungsgemäßen Verfahrens werden polymere und/oder in Polymeren eingebaute und/oder mit Polymeren verknüpfte α-Hydroxy- und/oder α-Sulfhydrylcarbonsäurederivate mit den, gegebenenfalls verkappten organischen Isocyanaten zu Hydantoingruppen enthaltenden Verbindungen umgesetzt.

Selbstverständlich können die erfindungsgemäßen Kondensationsprodukte, gegebenenfalls auch ihre Vorstufen, Polyestern, vorzugsweise Hydroxylgruppen enthaltenden Polyestern, z.B. vorzugsweise aus Maleinsäureanhydrid, Phthalsäureanhydrid und/oder Maleinsäure, Fumarsäure, Bernsteinsäure, Phthalsäure, Isophthalsäure, Terephthalsäure, Trimellithsäure und/oder Trimellithsäureanhydrid und/oder deren Ester, Ethylenglykol, Dipropylenglykol, Glycerin, Trimethylolpropan und/oder Trishydroxyethylisocyanurat und/oder der Kombination aus Cyanursäuretriarylestern und Polyolen, Polyethern, beispielsweise aus Ethylenoxid und/oder Bis-(hydroxyphenyl)-propan und Epichlorhydrin, Polyurethanen, Polyamiden, Polyolefinen, Polyacetaten, Polyepoxiden, Polyimiden, Polyamidimiden, Polyesterimiden, Polyesteramidimiden, Polyestern, Polyimino-Polyester, Polyimidisocyanaten, Polyhydantoinen, Polyhydantoinisocyanaten usw. zugemischt und gegebenenfalls an- und/oder einkondensiert oder die erfindungsgemäße Reaktion in Gegenwart dieser Komponenten durchgeführt werden. In allen Fällen entstehen dabei modifizierte Polymere, die neben den (Thio)-Hydantoinringen gegebenenfalls zusätzliche Ether-, Carbamidester, Carbonsäureester, Carbonsäure-amid-, imid-, -esteramid-, esterimid-, -amidimid- und/oder -esteramidimidgruppierungen enthalten.

Die Mengenverhältnisse dieser Zusatzstoffe können in weiten Grenzen schwanken, vorzugsweise werden bezüglich des erfindungsgemäßen Kondensates 10-500 Gew.-% eingesetzt.

Ferner können die Isocyanate in reiner Form oder als technische Reaktionsprodukte verwendet werden. Eine weitere Ausführungsform des erfindungsgemäßen Verfahrens beansprucht die Verwendung beliebiger Isocyanatgemische, wodurch beispielsweise in beliebigen Teilschritten jeweils andere organische Isocyanate eingesetzt werden können.

Im Rahmen des erfindungsgemäßen Verfahrens können Produkte mit gegebenenfalls verkappten Isocyanatgruppen entstehen. Durch Verwendung von Isocyanatgemischen, beispielsweise aus poly- und monofunktionellen Isocyanaten ist der Polymerisationsgrad und gegebenenfalls Isocyanatgehalt der erfindungsgemäßen Reaktion zu steuern. Demnach können Produkte mit vergleichbarem Polymerisationsgrad, aber geringerem, gegebenenfalls verkapptem Isocyanatgehalt durch Mitverwendung berechneter Anteile an Monoisocyanaten hergestellt werden. Geeignet sind beispielsweise Phenylisocyanat, α-Naphthylisocyanat, Isocyanatobenzoesäureester, Isocyanatoessigsäureester usw.

Die nach dem erfindungsgemäßen Verfahren zugänglichen nieder- bzw. monomolekularen Hydantoine besitzen biochemische Wirkungen, die erfindungsgemäßen Polyhydantoine eine besondere Temperaturbeständigkeit sowie eine gute Löslichkeit auch in vorzugsweise umweltfreundlichen Lösungsmitteln.

Die mit den erfindungsgemäßen Polykondensaten modifizierten Kunststoffe zeigen ebenfalls verbesserte Temperaturverhalten. Die Polymeren können zur Herstellung temperaturbeständiger Kleber, Lacke, Folien, Kunststoffe, Fasern und Pulver sowie zur Beschichtung hitzeresistenter Substrate verwendet werden. Ihre Eigenschaften können je nach Einsatzgebiet durch Zusatz von Füllstoffen, Pigmenten, nieder- und hochmolekularen Komponenten wie Polyurethane, Polyester, Polyesterimide, Polyimide, Polyamidimide, Polyamide, Polyesteramidimide, Polyvinylacetat, Epoxide, Polycyanate, Siliconharze in weiten Grenzen variiert werden.

Die Hydantoingruppen enthaltenden Produkte, die gegebenenfalls noch mit Amid-, Ester-, Imid-und/oder Isocyanuratgruppen modifiziert sind, sind vorzugsweise für Einbrennlacke geeignet. Diese Lackbindemittel kommen dabei im allgemeinen in Lösungsmitteln oder Lösungsmittelgemischen zum Einsatz, denen erfindungsgemäß zusätzlich ein gewisser Anteil Nichtlöser bzw. Verschnittmittel zugesetzt werden kann. Diese Verschnittmittel sind aliphatische, vorzugsweise aromatische Kohlenwasserstoffe, gegebenenfalls auch niedere Alkohole, beispielsweise Cyclohexan, Toluol, deren technische Gemische sowie Solvesso und Solventnaphtha, sowie Ethanol, Butanol, Aminoalkohol.

Der Festgehalt der möglichen Lacklösungen kann in weiten Grenzen schwanken und wird von Lösungsverhalten der Bindemittel, aber auch vom beabsichtigten Einsatzzweck bestimmt. Die Polyhydantoine besitzen selbst in vorzugsweise phenolfreien Lösemitteln eine ausgezeichnete Löslichkeit und können auf den üblichen Lackiermaschinen sowie als Tränklacke mit Festgehalten bis zu 60 %, gegebenenfalls auch aus der reinen Schmelze oder als Pulver, verarbeitet werden.

Schließlich wird auch die Härtungsreaktion des Beschichtungsmittels durch Zusatz der obengenannten Katalysatoren beschleunigt, d.h. die Reaktionszeit wird verkürzt bzw. die Einbrenntemperatur bei gleicher Zeit erniedrigt. Damit ist selbst auf besonders schnell laufenden Drahtlackiermaschinen eine vollständige Aushärtung des Lackfilms zu erzielen.

## Beispiel 1

190,2 g Apfelsäurediethylester werden unter $N_2$ ab 45 °C mit
262,7 g 4,4'-Diisocyanatodiphenylmethan vermischt und
mit
0,5 g Endoethylenpiperazin homogenisiert.

Man heizt in ca. 2 Stunden auf 160-170 °C und trägt dabei dann insgesamt 1 Mol $CO_2$ aus. Mit zunehmender Viskosität wird portionsweise mit insgesamt
954 g γ-Butyrolacton verdünnt.

Man heizt insgesamt ca. 1 Stunde auf 190 °C, ca. 4 Stunden auf 200-205 °C und ca. 1 Stunde auf 205 bis 210 °C.

Die ca. 30 %ige Lacklösung besitzt eine Viskosität von 58 750 $cP_{20\,°C}$ und wird zur Drahtlackierung mit γ-Butyrolacton auf ca. 22 % herunterverdünnt.

Das nach einer Methanolfällung verbleibende Bindemittel besitzt im IR-Spektrum die den Hydantoinen eigenen Banden.

Der in einem 4 m Ofen bei 10 m/Minute hergestellte 0,7 mm Cu-Lackdraht wurde nach DIN 46 453 geprüft und besitzt eine Erweichungstemperatur von mindestens 330 °C, einen Hitzeschock von mindestens 260 °C, eine Dauerwärmebeständigkeit von mindestens 21 Tagen bei 200 °C und trotz ausgezeichneter Elastizität eine Lackfilmhärte von mindestens 5 H. Die Chemikalienbeständigkeit ist gut.

## Beispiel 2

95,1 g Apfelsäurediethylester und
199,2 g N,N'-Bis-(2-methoxycarbonylpropyl-2-)-4,4'-diaminodiphenylmethan werden unter $N_2$ bei ca. 30 °C in
500 g m-Kresol gelöst, ab 35-40 °C mit der Lösung von
250,2 g 4,4'-Diisocyanatodiphenylmethan in
200 g Toluol versetzt, ca. 2 Stunden nachgerührt und
mit
0,5 g Endoethylenpiperazin versetzt. Anschließend heizt man über 150-170 °C unter Austragung von Toluol, Ethanol, $CO_2$ auf ca. 200 °C, hält ca. 3 Stunden bei 200-205 °C und weitere ca. 3 Stunden bei 205-210 °C.

Dabei muß im Zuge steigender Viskosität portionsweise mit insgesamt
590 g m-Kresol verdünnt und anschließend noch ca. 1 Stunde bei 200 °C nachgerührt werden.

Die ca. 30 %ige Polyhydantoinlösung besitzt eine Viskosität von ca. 98 000 $cP_{20\,°C}$.

Ein mit dieser Lacklösung in der Wärme beschichtetes und bei 250 °C während 15 Minuten und bei 300 °C während 10 Minuten eingebranntes Erichsen-Blech erhält einen Lackfilm von guter Elastizität, Haftfestigkeit und Chemikalienbeständigkeit sowie eine Bleistifthärte von 5 H.

Ein aus diesem Lack z.B. auf Glas in der bekannten Weise hergestellte Folie besitzt gute mechanische Eigenschaften, eine hohe Wärmebeständigkeit und einen Schmelzpunkt von mindestens 380 °C.

## Beispiel 3

76,8 g Trimellithsäureanhydrid und
8,3 g Isophthalsäure werden unter $N_2$ mit
150 g γ-Butyrolacton und

100 g Toluol vermischt, dann mit
142,6 g Apfelsäurediethylester versetzt und darauf ab 30-40 °C mit
362,8 g 4,4'-Diisocyanatodiphenylmethan verrührt.
Man rührt ca. 3 Stunden bei Raumtemperatur nach, versetzt dann bei ca. 30-45 °C mit der Lösung von
162,6 g 4,4'-Diisocyanatodiphenylmethan in
100 g Toluol, homogenisiert 1 Stunde bei 50 °C, fügt dabei
0,5 g Endoethylenpiperazin,
345,8 g Trimellithsäureanhydrid sowie
200 g Terephthalsäuredimethylester hinzu, homogenisiert, heizt dann unter Austragung, gegebenenfalls unter 200 Torr, von $CO_2$, Ethanol, Toluol usw. in Stufen über 120 °C, 150-170 °C auf 190 °C. Es werden insgesamt ca. 1 Stunde bei 190 °C, ca. 3 Stunden bei 205 bis 210 °C und ca. 2 Stunden bei 210 bis 215 °C bzw. bis zum Ende $CO_2$/Alkohol-Entwicklung kondensiert.
Danach wird das Gemisch bei ca. 170 °C mit
518,5 g Terephthalsäuredimethylester,
653,0 g Tris-(2-hydroxyethyl)-isocyanurat,
3,0 g Bleiacetat,
1,0 g Butyltitanat und
50 g Xylol versetzt, homogenisiert und dann in ca. 6 Stunden über 170 °C bei 200-220 °C soweit kondensiert, bis kein Destillat unter 150 °C mehr übergeht.
Schließlich versetzt man bei 170 °C mit
186 g Ethylenglykol und
46 g Glycerin, kondensiert nochmals bei 200-220 °C bis kein Destillat mehr unter 150 °C anfällt und kondensiert dann bei 210-230 °C zunächst direkt, schließlich unter ca. 200 Torr soweit, bis das mit γ-Butyrolacton auf ca. 50 % verdünnte Harz eine Viskosität von ca. 10 000 $cP_{20\,°C}$ erreicht hat.
Zum Drahtlackierversuch wurde der Ansatz bei 150-120 °C mit
1 000 g γ-Butyrolacton,
2 033 g Benzylalkohol verdünnt, mit der Lösung von
11 g Titantetrabutylat in
22 g Acetylaceton versetzt und ca. 1 Stunde bei 120-100 °C homogenisiert.
Die ca. 40 %ige Lacklösung besitzt eine Viskosität von 1 830 $cP_{20\,°C}$.
Der in einem 4 m Ofen bei 10 m pro Minute hergestellte 0,7 mm Cu-Lackdraht wurde nach DIN 46 453 geprüft. Er besitzt eine Erweichungstemperatur von mindestens 330 °C, einen Hitzeschock von mindestens 260 °C, eine Dauerwärmebeständigkeit von mindestens 14 Tagen bei 200 °C, eine Schabefestigkeit von mindestens 90 Doppelhüben, eine Filmhärte von mindestens 5 H, eine Durchschlagfestigkeit von mindestens 9 KV und eine gute Chemikalienbeständigkeit.

Andererseits kann das obige unverdünnte Bindemittel direkt in der bekannten Weise pulverisiert und als solches beispielsweise auf Erichsen-Blechen appliziert werden. Der nach einer Temperierung von 15 Minuten bei 250 °C und 10 Minuten bei 300 °C erhaltene Lackfilm besitzt ebenfalls die oben bereits angeführten guten mechanischen und elektrischen Eigenschaften, vor allem eine hohe Wärmebeständigkeit mit einem Schmelzpunkt oberhalb von 350 °C.

Beispiel 4

314,3 g Apfelsäuredibenzylester bei Raumtemperatur unter $N_2$ mit
217,7 g eines Gemisches aus 80 % 2,4- und 20 % 2,6-Toluylendiisocyanat vermischen und nach Zusatz von
0,5 g Endoethylenpiperazin in 2 Stunden auf 160-170 °C anheizen. Mit zunehmender Viskosität werden portionsweise insgesamt
651 g γ-Butyrolacton zugesetzt.
Man heizt insgesamt ca. 1 Stunde auf 190 °C, ca. 5 Stunden auf 200-205 °C und trägt dabei u.a. insgesamt ca. 1 Mol $CO_2$ aus.
Die ca. 40 %ige Oligohydantoinlösung besitzt eine Viskosität von 4 300 $cP_{20\,°C}$.
Sie wird mit
827 g Benzylalkohol,
534 g Benzoesäuremethylester und
267 g Propylencarbonat verdünnt und mit
1 085 g eines Polyesters aus 4,0 Mol Dimethylterephthalat, 0,8 Mol Trimellithsäureanhydrid, 0,9 Mol Terephthalsäure, 2,0 Mol Tris-(2-hydroxyethyl)-isocyanurat, 0,5 Mol Glycerin, 7,0 Mol Ethylenglykol, 200 g Solvesso, 3,0 g Bleiacetat und 1,0 g Butyltitanat mit einem Hydroxylgruppengehalt von ca. 4,5 Gew.-% versetzt.
Man homogenisiert das Gemisch 1 Stunde bei 180 bis 200 °C, gibt dann bei 100 °C bis 80 °C die Lösung von
15 g Titantetrabutylat in
30 g Acetylacetonat hinzu und rührt noch ca. 1 Stunde bei 80 bis 70 °C nach.

Die Viskosität der ca. 40 %igen Lacklösung beträgt 1 680 $cP_{20 °C}$.

Die mit ihr in einem 4 m Ofen bei 10 m pro Minute beschichteten Cu-Drähte von 0,7 mm Durchmesser besitzen eine Erweichungstemperatur von mindestens 330 °C, einen Hitzeschock von mindestens 260 °C und eine Dauerwärmebeständigkeit bei 200 °C von mindestens 14 Tagen sowie eine gute Chemikalienbeständigkeit.

Beispiel 5

307,0 g 4-Chlorphenylisocyanat und
246,3 g Apfelsäuredibutylester werden vermischt,
mit
0,2 g Endoethylenpiperazin versetzt und in ca. 2 Stunden auf ca. 200 °C angeheizt. Es werden ca. 7 Stunden bei 205-210 °C gerührt und dabei u.a. insgesamt ca. 1 Mol $CO_2$ ausgetragen. Gegebenenfalls kann bei zunehmender Viskosität mit γ-Butyrolacton, Acetophenon, Benzoesäureethylester und/oder Kresol verdünnt werden.

Nach Abschluß der Kondensation werden die gegebenenfalls verwendeten Lösungsmittel i.V. abdestilliert und der Rückstand aus beispielsweise Toluol umkristallisiert.

Die erhaltenen Kristalle haben einen FP von 127 °C. Aufgrund der IR- und NMR-Spektren sowie der Elementaranalyse :
ber. : $C_{21}$ 57,94 $H_{20}$ 4,63 $N_2$ 6,44 $CL_2$ 16,29
gef. : $C_{21}$ 57,9/58,0 $H_{20}$ 4,5 $N_2$ 6,3/6,65 $CL_2$ 16,1/16,2
handelt es sich um 1,3-Di-(4-chlorphenyl)-5-(n-butoxycarbonylmethylen)-hydantoin.

**Ansprüche**

1. Verfahren zur Herstellung von mindestens einen Hydantoinring oder Thiohydantoinring enthaltenden Verbindungen, dadurch gekennzeichnet, daß man mindestens 2 Mol von gegebenenfalls maskierten, organischen Iso(thio)cyanaten bei Temperaturen von −20 bis +500 °C mit einem Mol eines α-Hydroxy- und/oder α-Sulfhydrylcarbonsäurederivates der allgemeinen Formel I

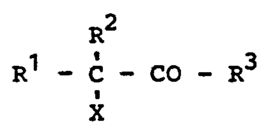

(I)

umsetzt, in der

$R^1$ und $R^2$ unabhängig voneinander Wasserstoff, ein Halogenatom, eine-CN-Gruppe, eine —COR-Gruppe mit R = Hydroxyl-, Amino-, Alkylamino-, Dialkylamino-, Alkoxy- oder Aroxygruppe, Wasserstoff, einen aliphatischen, cycloaliphatischen, aliphatisch-aromatischen, aromatischen oder heterocyclischen Rest; ein aliphatischer Rest, ein aliphatisch-aromatischer Rest, ein cycloaliphatischer Rest, ein aromatischer Rest, ein heterocyclischer Rest, die jeweils noch gegebenenfalls mit Halogen-, —$NO_2$-, —$COR^4$-, —CN-, —CO-, —OH-Gruppen mit $R^4$ = R substituiert oder auch gegebenenfalls gemeinsam bzw. jeder für sich mit $R^3$ als Glieder entsprechender cyclischer organischer Reste miteinander verknüpft sein können,

$R^3$ eine Hydroxyl-, Amino-, Alkylamino-, Dialkylamino-, Alkoxy- oder Aroxygruppe und

X eine OH- und SH-Gruppe bedeuten.

2. Verfahren nach Anspruch 1, dadurch gekennzeichnet, daß in der allgemeinen Formel I

$R^1$ und $R^2$ gleich oder verschieden, Wasserstoff, ein Halogenatom, eine —CN-Gruppe, eine —COR-Gruppe mit R = Hydroxyl-, Amino-, Alkylamino- mit $C_1$-$C_{20}$, Dialkylamino- mit jeweils $C_1$-$C_{20}$, Alkoxy- mit $C_1$-$C_{20}$ oder Aroxygruppe mit $C_6$-$C_{12}$, Wasserstoff, einem aliphatischen Rest mit $C_1$-$C_{20}$, aliphatischaromatischen Rest mit $C_7$-$C_{20}$, aromatischen Rest mit $C_6$-$C_{20}$ oder heterocyclischen Rest mit 4-16 Ringgliedern ;

$R^1$ und $R^2$ weiterhin ein aliphatischer Rest mit $C_1$-$C_{20}$, ein aliphatisch-aromatischer Rest mit $C_7$-$C_{20}$, ein aromatischer Rest mit $C_6$-$C_{20}$, ein heterocyclischer Rest mit 4-16 Ringgliedern, die jeweils noch gegebenenfalls mit Halogen-, $NO_2$-, $COR^4$-, CN-, —CO-, OH-Gruppen mit $R^4$ = R substituiert sein können und

$R^3$ eine Hydroxyl-, Amino-, Alkylamino mit $C_1$-$C_{20}$ Dialkylamino mit jeweils $C_1$-$C_{20}$, Alkoxy mit $C_1$-$C_{20}$ oder Aroxygruppe mit $C_6$-$C_{12}$ bedeuten.

3. Verfahren nach Ansprüchen 1 und 2, dadurch gekennzeichnet, daß als α-Hydroxy- oder α-Sulfhydrylverbindungen die Säure-, Ester- oder Amidderivate von Glykolsäure, Milchsäure, α-Hydroxypropionsäure, α-Hydroxybuttersäure, Atrolactinsäure, Mandelsäure, Benzilsäure, α-Hydroxy-β-phenylpionsäure, α-Hydroxy-γ-oxo-γ-phenylbuttersäure, α-Hydroxylävulinsäure, α-Hydroxy-γ-oxo-buttersäure, α-Hydroxy-β-oxo-buttersäure, 2,4-Dihydroxy-5-oxo-1,2-dihydrofuran, 3-Hydroxy-2-oxo-5-phenyl-tetrahydrofuran, Glycerinsäure, Tartronsäure, Apfelsäure, Weinsäure/Traubensäure, Citronensäure,

Ascorbinsäure oder α-Hydroxy-oxo-bernsteinsäure bzw. die entsprechenden Thioanaloga verwendet werden.

4. Verfahren nach Ansprüchen 1-3, dadurch gekennzeichnet, daß als organische Isocyanate Polyisocyanate verwendet werden.

5. Verfahren nach den Ansprüchen 1-4, dadurch gekennzeichnet, daß die α-Hydroxy- und/oder α-Sulfhydrylcarbonsäurederivate vor, während oder nach ihrem Umsatz mit organischen Isocyanaten mit Polycarbonsäuren, deren Anhydriden und/oder Ester und/oder Polyolen und/oder polyfunktionellen α-Aminocarbonsäurederivaten vermischt und umgesetzt werden.

6. Verwendung der nach den Ansprüchen 1 bis 5 erhaltenen (Thio)Hydantoinringe enthaltenden Verbindungen zur Herstellung von hitzebeständigen Überzugsmaterialien, Folien, Pulver, Kleber, Formkörper, Lacke, Fasern.

7. Verwendung nach Anspruch 6, dadurch gekennzeichnet, daß die (Thio)Hydantoinringe enthaltenden Verbindungen in Kombinationen mit Polyestern, Polyethern, Polyurethanen, Polyesteramidimiden, Polyamidimiden oder Polyimiden verwendet.

**Claims**

1. Process for the preparation of compounds containing at least one hydantoin ring or thiohydantoin ring, characterised in that at least 2 mol of optionally masked organic iso(thio)-cyanates are reacted at temperatures of from −20 to +500° with one mol of an α-hydroxy and/or α-sulphydrylcarboxylic acid derivative of the general formula I

$$R^1 - \underset{X}{\overset{R^2}{C}} - CO - R^3 \qquad (I)$$

in which

$R^1$ and $R^2$, independently of one another, represent hydrogen, a halogen atom, a CN group, a COR group, wherein R represents a hydroxyl, amino, alkylamino, dialkylamino, alkoxy or aroxy group or hydrogen or an aliphatic, cycloaliphatic, aliphatic-aromatic, aromatic or heterocyclic group ; or they may represent an aliphatic group, an aliphatic-aromatic group, a cycloaliphatic group, an aromatic group or a heterocyclic group, each of which may also be substituted by halogen, $NO_2$, $COR^4$, CN, CO, or OH groups, wherein $R^4$ = R, or they may also, either together or separately, be joined to $R^3$ as members of corresponding cyclic organic groups ;

$R^3$ represents a hydroxyl, amino, alkylamino, dialkylamino, alkoxy or aroxy group and

X represents an OH or SH group.

2. Process according to Claim 1, characterised in that in the general formula I

$R^1$ and $R^2$, which may be the same or different, each represents hydrogen, a halogen atom, a CN group, a COR group wherein R = hydroxyl, amino, $C_1$-$C_{20}$ alkylamino dialkylamino with in each case $C_1$-$C_{20}$, $C_1$-$C_{20}$ alkoxy or $C_6$-$C_{12}$ aroxy groups, hydrogen, a $C_1$-$C_{20}$ aliphatic group, a $C_7$-$C_{20}$ aliphatic-aromatic group, a $C_6$-$C_{20}$ aromatic group or a heterocyclic group having from 4 to 16 ring members ;

$R^1$ and $R^2$ may also represent a $C_1$-$C_{20}$ aliphatic group, a $C_7$-$C_{20}$ aliphatic-aromatic group, a $C_6$-$C_{20}$ aromatic group or a heterocyclic group having from 4 to 16 ring members, each of which may also be substituted with halogen, $NO_2$, $COR^4$, CN, CO or OH groups, wherein $R^4$ = R and

$R^3$ represents a hydroxyl, amino, $C_1$-$C_{20}$ alkylamino, dialkylamino with in each case $C_1$-$C_{20}$, $C_1$-$C_{20}$ alkoxy or $C_6$-$C_{12}$ aroxy group.

3. Process according to Claims 1 and 2, characterised in that the acid, ester or amide derivatives of glycollic acid, lactic acid, α-hydroxypropionic acid, α-hydroxybutyric acid, atrolactic acid, mandelic acid, benzylic acid, α-hydroxy-β-phenylpropionic acid, α-hydroxy-γ-oxo-γ-phenylbutyric acid, α-hydroxy-läevulinic acid, α-hydroxy-γ-oxo-butyric acid, α-hydroxy-β-oxo-butyric acid, 2,4-dihydroxy-5-oxo-1,2-dihydrofuran, 3-hydroxy-2-oxo-5-phenyl-tetrahydrofuran, glyceric acid, tartronic acid, malic acid, tartaric acid/racemic acid, citric acid, ascorbic acid, or α-hydroxy-oxo-succinic acid, and the corresponding thioanalogues are used as the α-hydroxy or α-sulphhydryl compounds.

4. Process according to Claims 1-3, characterised in that polyisocyanates are used as the organic isocyanates.

5. Process according to Claims 1-4, characterised in that the α-hydroxy and/or α-sulphhydryl carboxylic acid derivatives are mixed and reacted with polycarboxylic acids, or anhydrides and/or esters and/or polyols and/or polyfunctional α-aminocarboxylic acid derivatives thereof before, during or after the reaction thereof with organic isocyanates.

6. Use of the (thio)hydantoin-ring-containing compounds obtained according to Claims 1 to 5, for the production of heat-resistant coating materials, foils, powders, adhesives, moulded articles, lacquers and fibres.

7. Use according to Claim 6, characterised in that the compounds containing (thio)hydantoin rings are used in combinations with polyesters, polyethers, polyurethanes, polyester amidoimides, polyamidoimides or polyimides.

**Revendications**

1. Procédé de production de composés portant au moins un noyau d'hydantoïne ou de thiohydantoïne, caractérisé en ce qu'on fait réagir au moins 2 moles d'iso-(thio)cyanates organiques éventuellement bloqués à des températures de −20 à +500 °C avec 1 mole d'un dérivé d'acide α-hydroxy- et/ou α-sulfhydrylcarboxylique de formule générale I

$$R^1 - \overset{\displaystyle R^2}{\underset{\displaystyle X}{C}} - CO - R^3 \qquad (I)$$

dans laquelle :

$R^1$ et $R^2$ représentent, indépendamment l'un de l'autre, de l'hydrogène, un atome d'halogène, un groupe —CN, un groupe —COR où R = un groupe hydroxyle, amino, alkylamino, dialkylamino, alkoxy ou aroxy, l'hydrogène, un reste aliphatique, cycloaliphatique, aliphatique-aromatique, aromatique ou hétérocyclique ; un reste aliphatique, un reste aliphatique-aromatique, un reste cycloaliphatique, un reste aromatique, un reste hétérocyclique qui peuvent encore être substitués chacun, le cas échéant, avec un halogène, des groupes —$NO_2$, —$COR^4$, —CN, —CO, —OH, $R^4$ étant égal à R, ou qui peuvent aussi être liés éventuellement ensemble ou chacun pour soi avec $R^3$ comme membres de restes organiques cycliques correspondants,

$R^3$ est un groupe hydroxyle, amino, alkylamino, dialkylamino, alkoxy ou aroxy et

X désigne les groupes OH et SH.

2. Procédé suivant la revendication 1, caractérisé en ce que dans la formule générale I

$R^1$ et $R^2$, égaux ou différents, représentent de l'hydrogène, un atome d'halogène, un groupe —CN, un groupe —COR avec R = un groupe hydroxyle, amino, alkylamino en $C_1$ à $C_{20}$, dialkylamino en $C_1$ à $C_{20}$ pour chaque radical, alkoxy en $C_1$ à $C_{20}$ ou aroxy en $C_6$ à $C_{12}$, l'hydrogène, un reste aliphatique en $C_1$ à $C_{20}$, un reste aliphatique-aromatique en $C_7$ à $C_{20}$, un reste aromatique en $C_6$ à $C_{20}$ ou un reste hétérocyclique de 4 à 16 chaînons dans le noyau ;

$R^1$ et $R^2$ désignent en outre un reste aliphatique en $C_1$ à $C_{20}$, un reste aliphatique-aromatique en $C_7$ à $C_{20}$, un reste aromatique en $C_6$ à $C_{20}$, un reste hétérocyclique ayant 4 à 6 chaînons dans le noyau, qui peuvent être substitués chacun, le cas échéant, avec un halogène, des groupes $NO_2$, $COR^4$, CN, —CO, OH, $R^4$ étant égal à R et

$R^3$ est un groupe hydroxyle, amino, alkylamino en $C_1$ à $C_{20}$, dialkylamino en $C_1$ à $C_{20}$ dans chaque radical, alkoxy en $C_1$ à $C_{20}$ ou aroxy en $C_6$ à $C_{12}$.

3. Procédé suivant les revendications 1 et 2, caractérisé en ce qu'on utilise comme composés α-hydroxylés ou α-sulfhydrylés, les dérivés d'acides d'esters ou d'amides de l'acide glycolique, de l'acide lactique, de l'acide α-hydroxypropionique, de l'acide α-hydroxybutyrique, de l'acide atrolactique, de l'acide mandélique, de l'acide benzilique, de l'acide α-hydroxy-β-phénylpropionique, de l'acide α-hydroxy-γ-oxo-γ-phénylbutyrique, de l'acide α-hydroxylévulinique, de l'acide α-hydroxy-γ-oxobutyrique, de l'acide α-hydroxy-β-oxobutyrique, du 2,4-dihydroxy-5-oxo-1,2-dihydrofuranne, du 3-hydroxy-2-oxo-5-phényltétrahydrofuranne, de l'acide glycérique, de l'acide tartronique, de l'acide malique, de l'acide tartrique droit/tartrique racémique, de l'acide citrique, de l'acide ascorbique ou de l'acide α-hydroxy-oxo-succinique et les analogues thio correspondants.

4. Procédé suivant les revendications 1 à 3, caractérisé en ce qu'on utilise comme isocyanates organiques des polyisocyanates.

5. Procédé suivant les revendications 1 à 4, caractérisé en ce que les dérivés d'acides α-hydroxy- et/ou α-sulfhydrylcarboxyliques sont mélangés et amenés à réagir avant, pendant ou après leur réaction avec des isocyanates organiques, avec des acides polycarboxyliques, leurs anhydrides et/ou esters et/ou des polyols et/ou des dérivés d'acides α-aminocarboxyliques polyfonctionnels.

6. Utilisation des composés contenant des noyaux de (thio)hydantoïne obtenus suivant les revendications 1 à 5 pour la production de matières de revêtement, feuilles, poudres, colles, pièces moulées, peintures et fibres stables à la chaleur.

7. Utilisation suivant la revendication 6, caractérisée en ce que les composés contenant des noyaux de (thio)hydantoïne sont utilisés en associations avec des polyesters, des polyéthers, des polyuréthannes, des polyester-amide-imides, des polyamide-imides ou des polyimides.